(19) Europäisches Patentamt
European Patent Office
Office européen des brevets

(11) Veröffentlichungsnummer: **0 060 222**
**B1**

(12) # EUROPÄISCHE PATENTSCHRIFT

(45) Veröffentlichungstag der Patentschrift: **06.12.89**

(51) Int. Cl.⁴: **C 07 D 249/08**

(21) Anmeldenummer: **82810097.4**

(22) Anmeldetag: **04.03.82**

(54) **Halogenmethyltriazole.**

(30) Priorität: **10.03.81 CH 1622/81**

(43) Veröffentlichungstag der Anmeldung: **15.09.82 Patentblatt 82/37**

(45) Bekanntmachung des Hinweises auf die Patenterteilung: **06.12.89 Patentblatt 89/49**

(84) Benannte Vertragsstaaten: **AT BE CH DE FR GB IT LI NL**

(56) Entgegenhaltungen:
**CHEMICAL ABSTRACTS, Band 93, Nr. 5, 4. August 1980, Seite 920, Nr. 46530w, Columbus, Ohio, USA M.S. PEVZNER et al.: "Heterocyclic nitro compounds. 25. l-Hydroxymethyl-3-nitro-1,2,4-triazoles and their derivatives" CHEMISTRY OF HETEROCYCLIC COMPOUNDS Vol. 16 (1980) Seiten 189-194.**

(73) Patentinhaber: **CIBA- GEIGY AG, Klybeckstrasse 141, CH- 4002 Basel (CH)**

(72) Erfinder: **Kunz, Walter, Dr., Im Goldbrunnen 55, CH- 4104 Oberwil (CH)**
Erfinder: **Maier, Ludwig, Dr., Im Lee 28, CH- 4144 Arlesheim (CH)**

**EP 0 060 222 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue 1-Halogenmethyl-1H-1,2,4-triazole sowie ein Verfahren zu ihrer Herstellung.

Die erfindungsgemässen 1-Halogenmethyl-1H-1,2,4-triazole entsprechen der allgemeinen Formel I

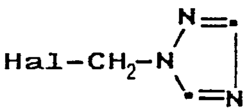

(I),

worin Hal für Chlor, Brom und Jod steht.

Die Verbindungen der Formel I werden als Ausgangsverbindungen zur Herstellung von Triazolylmethyl-phosphonaten der Formel II

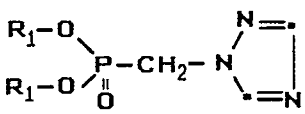

(II),

worin die Reste $R_1$ unabhängig voneinander Phenyl oder $C_1$-$C_4$-Alkyl bedeuten, oder Triazolylmethyl-phosphoniumverbindungen der Formel III,

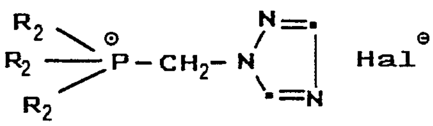

(III)

worin die Reste $R_2$ unabhängig voneinander Phenyl oder gegebenenfalls durch Hydroxyl substituiertes $C_1$-$C_4$-Alkyl bedeuten und Hal die unter Formel I gegebene Bedeutung hat, verwendet.

Diese Verbindungen dienen ihrerseits als Zwischenprodukte für die Herstellung von pflanzenfungiziden Triazol-styrolen der Formel IV,

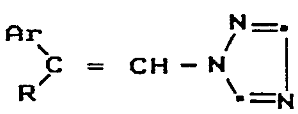

(IV)

worin

Ar einen gegebenenfalls durch $C_1$-$C_4$-Alkyl, $C_1$-$C_4$-Alkoxy, Cyan, Nitro, Trifluormethyl oder ein bis drei Halogenatome substituierten Phenylrest bedeutet und
R für Wasserstoff; $C_1$-$C_{10}$-Alkyl; $C_2$-$C_5$-Alkenyl; $C_3$-$C_8$-Cycloalkyl; gegebenenfalls durch $C_3$-$C_8$-Cycloalkyl oder gegebenenfalls durch Halogen, $C_1$-$C_4$-Alkyl oder $C_1C_4$-Alkoxy substituiertes Phenyl substituiertes $C_1$-$C_4$-Alkyl steht, und pflanzenfungiziden 1-(2-Phenyläthyl)-triazolen der Formel V,

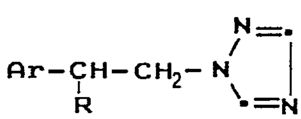

(V),

worin Ar und R die unter Formel IV angegebene Bedeutung haben.

Die Verbindungen II, III und IV sowie das Herstellungsverfahren von Verbindungen V sind Gegenstand der publizierten europäischen Patentanmeldung Nr. 0 063 099. Die Verbindungen der Formel V sind in der deutschen Offenlegungsschrift 2 735 872 beschrieben.

Die bevorzugte Verbindung der Formel I, gemäss vorliegender Erfindung ist 1-Chlormethyl-1H-1,2,4-triazol.

Die Verbindungen der Formel I der vorliegenden Erfindung werden nach dem erfindungsgemässen Verfahren hergestellt, indem man 1-Hydroxy-methyl-1H-1,2,4-triazol der Formel VI

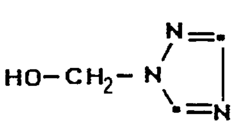

(VI)

in Abwesenheit von Basen mit einem Halogenierungsmittel umsetzt und die entstandenen Hydrohalogenide der Formel VII,

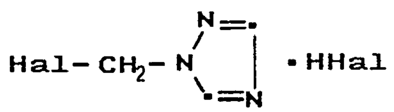

(VII),

worin Hal die unter Formel I angegebene Bedeutung hat, mit einem anorganischen Hydroxid behandelt.

Als Basen eignen sich starke anorganische Hydroxide wie Natrium- und Kaliumhydroxid.

Als Halogenierungsmittel können Verwendung finden: Phosgen, Thionylchlorid, Thionylbromid, Phosphortrichlorid, Phosphortribromid, Phosphorpentachlorid, Phosphoroxychlorid oder Jodwasserstoffsäure. Vorzugsweise werden Thionylchlorid und Thionylbromid zur Halogenierung eingesetzt, da die entstehenden Nebenprodukte gasförmig sind und deshalb die Reaktion nicht beeinflussen. Die Jodverbindungen werden mit Vorteil aus bereits halogenierten Verbindungen erhalten, wenn man diese mit Jodwasserstoffsäure umsetzt.

Die Halogenierungsreaktion wird in der Regel in inerten Lösungsmitteln ausgeführt, wie z. B. Kohlenwasserstoffen wie Hexan, Cyclohexan, Benzol, Toluol oder Xylol oder Äthern wie Diäthyläther, Tetrahydrofuran, Dioxan oder Dimethoxyäthan. Bei Verwendung von flüssigen Halogenierungsmitteln kann häufig ganz auf ein Lösungsmittel verzichtet werden. Die Reaktion wird dann in einem Überschuss der Reagenzes, z. B. Thionylchlorid oder -bromid durchgeführt.

Die 1-Halogenmethyl-1H-1,2,3,4-triazole der Formel I sind bei Raumtemperatur und auch bei 0°C instabile Verbindungen. Jedoch wurde überraschenderweise festgestellt, dass Lösungen von 1-Halogenmethyl-1H1,2,4-triazolen der Formel I in Acetonitril monatelang ohne Zersetzung haltbar sind.

Die Ausgangsverbindung der Formel VI ist aus Khim. Geterosikl, Soedi., 1980, 251 (Engl. Übersetzung: Chem. Heterocycl. Comp. 1980, 16 189) bekannt. In dem dort angegebenen Verfahren wird das Hydroxymethyl-triazol unter Verwendung von wässriger Formalinlösung hergestellt.

Das 1-Hydroxamethyl-1H-1,2,4-triazol kann vorteilhaft hergstellt werden, indem man in nicht-wässrigem Medium 1H-1,2,4-Triazol in Gegenwart von katalytischen Mengen Base mit Paraformaldehyd umsetzt.

Die anschliessenden Beispiele dienen der näheren Illustration der Erfindung. Temperaturen sind in Celsiusgraden, °C, Drucke in Millibar, mb, angegeben.

**Beispiel 1: Herstellung von 1-Chlormethyl-1H-1,2,4-triazol-hydrochlorid**

Zu 600 ml Thionylchlorid lässt man innerhalb von 1,5 Stunden eine Schmelze von 195,3 g (1.74 Mol) 1-Hydroxymethyl-1H-1,2,4-triazol zutropfen. Es setzt eine starke Gasentwicklung ein und die Reaktionsmischung beginnt zu sieden. Nachdem die Schmelze vollständig zugesetzt worden ist, wird unter Kochen am Rückfluss noch für 2 Stunden gerührt. Nach dem Abkühlen wird das ausgefallene, gelbe 1-Chlormethyl-1H-1,2,4-triazol-hydrochlorid abfiltriert und zweimal mit je 300 ml Diäthyläther gewaschen. Ausbeute 272 g (93,5 %), Smp. 120 - 127°.

$C_3H_4ClN_3$ HCl (154)

Ber.: C 23,40 %; H 3,27 %; N 27,29 %
Gef.: C 23,40 %; H 3,5 %; H 27,30 %,

$^1$H-NMR ($CD_3OD$): δ = 9,77 (s; IH, 5-CH); 8,55 (s; IH, 3-CH); 5,9 (s; 2H, $CH_2$-Cl) und 5,06 (s; IH, HCl) ppm.

**Beispiel 2: Herstellung von 1-Chlormethyl-1H-1,2,4-triazol**

Zu 240,6 g (1.44 Mol) 1-Chlormethyl-1H-1,2,4-triazol-hydrochlorid in einem Gemisch von 600 ml Wasser und 400 ml Chloroform lässt man unter kräftigem Rühren 80 g Natriumhydroxid in 250 ml Wasser zutropfen. Nach Sättigen mit Natriumchlorid wird dreimal mit Chloroform extrahiert. Durch Trocknen, Eindampfen der Chloroformextrakte und fraktionierte Destillation des Rückstandes erhält man 156,2 g (83.05 %) 1-Chlormethyl-1H-1,2,4-triazol, Sdp. 52 - 54 % / 0.27 mb.

$^1$H-NMR ($CDCl_3$): δ = 8,8 (s; 1H, 5-CH); 8,3 (s; 1H, 3-CH) und 6,3 (s; 2H, $CH_2Cl$) ppm.

**Patentansprüche**

1. 1-Halogenmethyl-1H-1,2,4-triazole der allgemeinen Formel I,

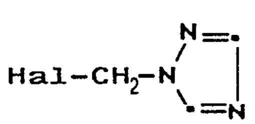

(I),

worin Hal für Chlor, Brom und Jod steht.

2. Die Verbindung 1-Chlormethyl-1H-1,2,4-triazol gemäss Anspruch 1.

3. Verfahren zur Herstellung der Verbindungen der Formel I, gemäss Anspruch 1, durch Umsetzung von 1-Hydroxymethyl-1H-1,2,4-triazol der Formel VI

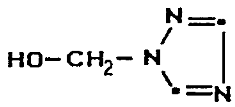

(VI)

mit einem Halogenierungsmittel dadurch gekennzeichnet, dass man die Umsetzung in Abwesenheit von Basen durchführt und die entstandenen Hydrohalogenide der Formel VII,

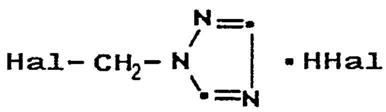

(VII),

worin Hal die unter Formel I, im Anspruch 1, angegebene Bedeutung hat, mit einem anorganischen Hydroxid behandelt.

4. Verfahren gemäss Anspruch 3 zur Herstellung von 1-Chlormethyl-1H-1,2,4-triazol, dadurch gekennzeichnet, dass man als Halogenierungsmittel Thionylchlorid verwendet.

**Claims**

1. 1-halomethyl-1H-1,2,4-triazoles of the general formula I

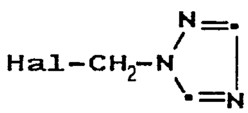

(I),

wherein Hal is chlorine, bromine and iodine.

2. The compound 1-chloromethyl-1H-1,2,4-triazole according to claim 1.

3. A process for the production of the compounds of the formula I according to claim 1, by reacting 1-hydroxy-methyl-1H-1,2,4-triazole of the formula VI

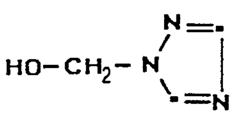

(VI)

with a halogenating agent, which process comprises effecting the reaction in the absence of bases and treating the resultant hydrohalides of the formula VII

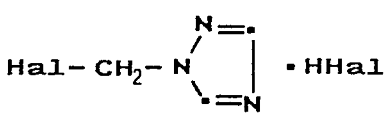

(VII),

wherein Hal is as defined for formula I in claim 1, with an inorganic hydroxide.

4. A process according to claim 3 for the production of 1-chloromethyl-1H-1,2,4-triazole, wherein the halogenating agent used is thionyl chloride.

(I),

**Revendications**

1. 1-halogénométhyl-1H-1,2,4-triazoles de formule générale I

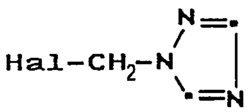

(I),

où Hal représente le chlore, le brome et l'iode.

2. Le composé 1-chlorométhyl-1H-1,2,4-triazole, selon la revendication 1.

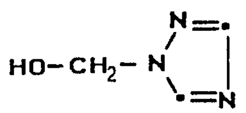

(VI),

3. Procédé de préparation des composés de formule I selon la revendication 1, par réaction du 1-hydroxyméthyl-1H-1,2,4-triazole de formule VI

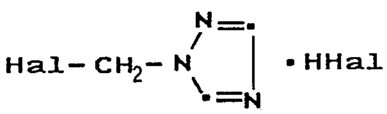

(VII),

avec un agent d'halogénation, caractérisé en ce que l'on effectue la réaction en l'absence de bases et en ce que l'on traite les hydrohalogénures formés de formule VII
où Hal a la signification indiquée sous la formule I dans la revendication 1, avec un hydroxyde minéral.

4. Procédé selon la revendication 3 pour la préparation du 1-chlorométhyl-1H-1,2,4-triazole, caractérisé en ce que l'on utilise, comme agent d'halogénation, le chlorure de thionyle.